# EUROPÄISCHE PATENTSCHRIFT

(11) **EP 0 440 146 B1**
(45) Veröffentlichungstag und Bekanntmachung des Hinweises auf die Patenterteilung: **20.11.1996**
(21) Anmeldenummer: 91101094.0
(22) Anmeldetag: 28.01.1991
(51) Int. Cl.: C12N 15/13, C12P 21/08, C07K 16/00, G01N 33/53

(54) **Herstellung und Verwendung von Genbanken synthetischer menschlicher Antikörper ("synthetische Human-Anti-Körper-Bibliotheken)**
Preparation and use of synthetic human antibody gene bank (synthetic human antibody libraries)
Préparation et utilisation de banques de gènes d'anticorps synthétiques humains (bibliothèques d'anticorps humains synthétiques)

(30) Priorität: 01.02.1990 DE 4002897; 09.02.1990 DE 4003880
(43) Veröffentlichungstag der Anmeldung: 07.08.1991
(73) Patentinhaber: BEHRINGWERKE Aktiengesellschaft, 35001 Marburg (DE)
(72) Erfinder: Little, Melvyn, Dr., W-6903 Neckargemünd (DE); Breitling, Frank Berthold, W-6908 Wiesloch (DE); Seehaus, Thomas, Dr., W-6148 Heppenheim (DE); Dübel, Stefan, W-6900 Heidelberg (DE); Klewinghaus, Iris, W-6800 Mannheim (DE)

(56) Entgegenhaltungen:
- WO-A-88/06630
- PROTEIN ENGINEERING, Band 2, Nr. 4, 1988, Eynsham, Oxford (GB); I.S. DUNN et al., Seiten 283-291

## Beschreibung

Die Erfindung betrifft die Herstellung und Verwendung von Genbanken synthetischer menschlicher Antikörper (huAK) oder Antikörperteile, die die antigenbindende Domäne enthalten. Ausgehend von einem huAK-Gerüst in einem geeigneten Vektor werden die hypervariablen Bereiche der Antikörper cDNA durch annähernd "zufällig" ("random") zusammengesetzte Oligonukleotide gebildet. Relativ konservierte Aminosäuren in den hypervariablen Bereichen sind dabei durch die Wahl entsprechender Nukleotide während der Oligonukleotidsynthese berücksichtigt, ebenso wie das Verhältnis der eingesetzten Nukleotide zueinander so vorgegeben ist, daß ein "Nonsense"-Codon nur in höchstens jeder 89. Position zu erwarten ist. Durch Expression dieser synthetischen huAK cDNA in mikrobiellen Expressionssystemen, z.B. dem nachstehend beschriebenen Vektor pFMT in E.coli, steht somit eine synthetische huAK-Bibiothek mit einem umfassenden Repertoire zum Durchprüfen ("Screenen") mit ausgewählten Antigenen **in vitro** zur Verfügung.

Es wird geschätzt, daß das Immunsystem des Menschen bzw. eines Säugetiers zwischen 10⁶ und 10⁸ verschiedene Antikörper besitzt. Diese Zahl von Antikörpern reicht offenbar aus, um eine Immunreaktion des Körpers sowohl gegen sämtliche in der Natur vorkommende Antigene als auch gegen künstliche Antigene hervorzurufen. Wenn man weiterhin in Betracht zieht, daß oft unterschiedliche Antikörper mit demselben Antigen reagieren, wird das Repertoire an wirklich verschiedenen Antikörpern eher im Bereich 10⁶ bis 10⁷ anzusiedeln sein.

Bisher werden spezifische Antikörper stets ausgehend von einer Immunisierung mit dem jeweiligen Antigen erhalten, beispielsweise Injektion des Antigens in den Organismus oder Inkubation von Milzzellen **in vitro** mit diesem Antigen. Im Fall von polyklonalen Antikörpern lassen sich die Immunglobuline anschließend aus dem Serum isolieren und z.B. durch Absorptionsverfahren die spezifischen Antikörper daraus gewinnen. Monoklonale Antikörper werden aus den Zellüberständen bzw. dem Zell-Lysat von mit einzelnen B-Lymphozyten fusionierten, klonierten Milztumorzellen (Hybridomzellen) isoliert. Die oben geschilderten Verfahren sind insbesondere zur Herstellung von spezifischen Human-Antikörpern bzw. humanen monoklonalen Antikörpern nicht geeignet.

Die vorliegende Erfindung stellte sich deshalb die Aufgabe, eine allgemein gangbare Methode zur Erzeugung spezifischer humaner monoklonaler Antikörper (huMAK's) oder Antikörperteile zu entwickeln, die synthetische hypervariable Domänen enthalten.

Es wurde gefunden, daß durch die Verwendung von annähernd zufällig ("random") synthetisierten Oligonukleotiden kodierend für die jeweils drei hypervariablen Bereiche der variablen Teile von schwerer bzw. leichter Ketten (genannt CDR1, 2 und 3 wobei CDR "complementary determining region" bedeutet) synthetische humane Genbanken erzeugt werden können. Die synthetisierte Antikörper-DNA wurde dann, vorzugsweise nach Amplifizierung mit der Polymerase-Ketten-Reaktion (PCR), bevorzugt in einen besonders dafür konstruierten Antikörper-Expressionsvektor ligiert, nämlich den Vektor pFMT.

Eine Zusammenstellung der Oligonukleotide, die für die Synthese der variablen Domänen von schweren und leichten Ketten eingesetzt werden, zeigt Tab. 1. Satz A enthält dabei weniger Einschränkungen als Satz B. Folgende Einschränkungen der Zufälligkeit bei der Synthese hypervariabler Bereiche (siehe CDR-Regionen in Tab. 4) wurden in H3, H4, H6, L2, L3 und L5 bei Satz A vorgenommen, um erstens Sequenzpositionen für bestimmte konservierte Aminosäuren zu berücksichtigen, zweitens die Zahl der Stopcodons zu reduzieren und drittens eine neue Restriktionsstelle einzubauen.
(a) Zur Verringerung der Wahrscheinlichkeit des Auftretens von Stopcodons wurde jeweils an der ersten Stelle jedes Codons nur die Hälfte der Menge der drei anderen Nukleotide bei T vorgegeben und an der dritten Position jedes Codons wurde A weggelassen. Damit ist im statistischen Mittel nur jedes 89. Codon ein Stopcodon.
(b) Für das 2. Codon in der CDR1-Region der leichten Kette wurden nur Nukleotide zugelassen, die für die Aminosäuren V, A oder G kodieren.
(c) Ebenso wurde für Codon Nr. 10 in der CDR1 Region der leichten Kette und für Codon Nr. 4 in der CDR1 Region der schweren Kette nur die Kombinationen zugelassen, die für V, I oder M kodieren.
(d) In der CDR3 Region der leichten Kette wurden für Codon Nr. 1 nur die Nukleotide zugelassen, die für die Aminosäure Glutamin kodieren.
(e) In der CDR2 Region der schweren Kette werden für Codon Nr. 11 nur die Nukleotide zugelassen, die für die Aminosäure Tyrosin kodieren.
(f) Zweckmäßigerweise wurde zur Einführung einer Restriktionsschnittstelle für MluI an der dritten Stelle des letzten Codons in der CDR2 Region der schweren Kette ein A eingebaut.

Vorzugsweise wurde die Zufälligkeit dieser Oligonukleotide noch weiter eingeschränkt in den Positionen, wo überwiegend eine oder wenige Aminosäuren vorkommen (Satz B von Tab. 1, die Einschränkungen beruhen hier auf den Tabellen von Kabat et al. (1987), Sequences of Proteins of Immunological Interest-U.S. Dept. of Health and Human Services, U.S. Government Printing Offices). Eine Liste der entsprechenden Nukleotide und kurze Erläuterungen zu den Codonzusammensetzungen sind in Tab. 1 und den Erläuterungen zu Tab. 1 zusammengefaßt.

Nach Ligation gleicher Molmengen von Oligonukleotiden H1 bis H7 bzw. L1 bis L5 werden diese in den vorbereiteten Expressionsvektor pFMT ligiert. Vorzugsweise sollte ein PCR-Schritt mit den "Primern" H1 und H8 bzw. L1 und L6 vorgeschaltet werden, um die Menge von DNA zu amplifizieren. Nach Herstellung von geeigneten Restriktionsschnittstellen mit passenden Restriktionsenzymen an den Enden der Antikörper-DNA wird die DNA in den Antikörper-Expressionsvektor pFMT in gleicher Weise wie oben ligiert (siehe Beispiele).

Der Expressionsvektor pFMT ermöglicht die Expression von Antikörper-cDNA und anschließende Sezernierung der Expressionsprodukte in Bakterien (E.coli). Das Antikörper-Operon des Plasmids enthält die Sequenzen der variablen Teile sowohl der schweren als auch der leichten Kette eines Antikörpers. Geeignete "Leader"-Sequenzen aus dem aminoterminalen Teil eines bakteriellen Proteins ermöglichen die Sezernierung der Antikörperteile. Die "Leader"Sequenzen werden bei der Sekretion von einem bakteriellen Enzym abgespalten. Während der Sekretion der Antikörper-cDNA-Produkte assoziieren die leichten und schweren Ketten des Antikörpers (mit oder ohne angrenzende konstante Domäne). Dadurch wird ein Antikörper oder Antikörperfragment gebildet, der bzw. das eine funktionelle Antigen-Bindungsstelle enthält. Ähnliche Konstrukte für einzelne Antikörper sind auch von anderen Autoren beschrieben worden (Better et al. (1988), Science 240, 1041, und Skerras & Plückthun (1988), Science 240, 1038).

In der durch die Expression in beispielsweise E.coli entstandenen synthetischen Human-Antikörper-Bibliothek werden die gesuchten humanen Antikörper bzw. Antikörperteile durch Screenen von bakteriellen Klonen mit dem Antigen der Wahl aufgefunden. In einer bevorzugten Ausführungsform wird zusätzlich eine Sequenz eingebaut, die für ein Markerpeptid kodiert, z.B. eine "TAG"-Sequenz, so daß die Expressionsprodukte mit etablierten monoklonalen Antikörpern gegen das Markerpeptid (Wehland et al. (1984), EMBO J. 3, 1295) auf einfache Weise nachgewiesen werden können.

Die vorgenannten beispielhaften Formulierungen und die nachfolgenden Beispiele sollen so verstanden werden, daß sie die Erfindung erläutern, aber nicht einschränken.

Die Erfindung betrifft folglich Genbanken synthetischer huAK oder deren antigenbindenden Teile erhalten durch (1) zufällig erzeugte cDNA für die hypervariablen Regionen wobei die Zufallssequenzen durch die Punkte (a) bis (e) Satz A bzw. gemäß Tab. 1, Satz B eingeschränkt sind, (2) daran anschließend vorzugsweise einen Amplifikationsschritt dieser Zufallsequenzen und (3) Ligierung der genannten cDNA in einen geeigneten Expressionsvektor, vorzugsweise pFMT, wobei in einer bevorzugten Ausführungsform zusätzlich eine für ein Markerpeptid kodierende Sequenz eingebaut wird.

Ferner betrifft die Erfindung Verfahren zur Herstellung o.g. Genbanken und Verfahren sowie deren Verwendung zur Isolierung von Klonen, die spezifische Antikörper bzw. deren antigenbindenden Teile sezernieren.

Die Erfindung ist schließlich in den Beispielen im einzelnen ausgeführt und in den Patentansprüchen enthalten.

### Beispiele:

### Beispiel 1: Herstellung eines Antikörper-Expressionsvektors

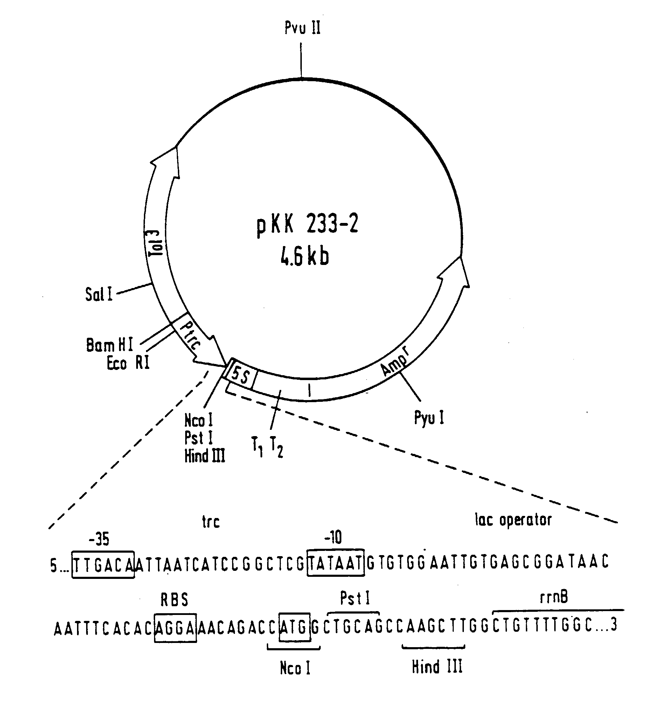

Das Plasmid pKK233-2 (Amann und Brosius, (1985) Gene 40, und Straus und Gilbert, (1985) Proc. Natl. Acad. Sci. 82, 2014) wurde als Basisvektor für den Aufbau des Antikörper-Expressionsvektors gewählt (Fig. 1).

Vor dem Einbau des Antikörper-Operons wurde das Plasmid mit SalI und BamHI geschnitten, die Enden mit Klenow-Polymerase aufgefüllt und ligiert. Dadurch wurden diese beiden Restriktionsstellen und die dazwischenliegende DNA entfernt. Außerdem wurde das Plasmid mit HindIII gespalten, die Enden mit der Klenow-Polymerase aufgefüllt und mit BamHI-Linkern ligiert. Durch diese Operation wurde die HindIII-Restriktionsstelle entfernt und eine BamHI-Stelle insertiert. In dieses modifizierte Plasmid wurde die Antikörper-DNA insertiert. Ein vereinfachter Aufbau des Antikörper-Operons, das für eine dicistronische Antikörper-mRNA kodiert, wird in Tab. 2 gezeigt. Um die Sezernierung des Antikörpers zu ermöglichen, wurde die "Leader"-Sequenz des bakteriellen Enzyms Pektatlyase verwendet. Die "Leader"-Sequenz von diesem Enzym ist schon zur Expression und Sezernierung eines chimären Maus-Mensch-Antikörpers (Fab-Fragment, Better et al., a.a.O.) sowie des variablen Teils eines "humanisierten" Antikörpers (Ward et al., a.a.O.; Huse et al., a.a.O.) verwendet worden. DNA für die erste "Leader"Sequenz (P₁ vor der schweren Kette) und die Sequenz für eine zweite Ribosomenbindungsstelle (RBS) und eine zweite "Leader"-Sequenz (P₂ vor der leichten Kette) wurden aus mehreren Oligonukleotiden synthetisiert (Tab. 3).

Antikörper cDNAs, die für die variable Regionen der schweren und leichten Ketten eines menschlichen Antikörpers (HuVhlys bzw. HuVllys; Riechmann et al., (1988) J. Mol. Biol. 203, 825) kodieren, wurden von Dr. G. Winter (Cambridge, UK) erhalten. Die Restriktionsstellen HindIII (HuVhlys) und EcoRV (HuVllys) wurden eingeführt, um die Insertion der Antikörper-cDNA in den Expressionsvektor zu ermöglichen. Weitere Restriktionsstellen für BanII (HuVhlys) und BstEII bzw. KpnI (HuVllys) wurden eingeführt, um hypervariable Bereiche "en bloc" umzutauschen. Am Ende der HuVhlys-cDNA-Sequenz wurde ein Stopsignal eingebaut. Eine BanII-Stelle in der leichten Kette wurde entfernt. Diese Änderungen wurden mittels "site directed mutagenesis" in dem Bakteriophagen M13mp18 durchgeführt (Zoller und Smith, Meth. Enzymol. 100, 468-500). Die Sequenz der fertigen Antikörper-DNA ist in Tab. 4 gezeigt.

Für die Insertion der "Leader"-Sequenz P₁ (Tab. 3) wurde das modifizierte Plasmid pKK233-2 mit den Restriktionsenzymen NcoI und PstI verdaut und P₁ zwischen diesen Stellen insertiert (pKK233-2-P₁). Weitere Klonierungsschritte bis auf den letzten Schritt wurden mit dem Plasmid pUC18 unternommen. Der Grund besteht darin, daß die Anwesenheit von einzelnen Teilen des Antikörper-Operons in dem Expressionsvektor das Wachstum des bakteriellen Wirts beeinträchtigt.

Vor der Klonierung in pUC18 mußte dessen BamHI-Restriktionsstelle entfernt werden. Nach einem Verdau mit BamHI wurden die einzelsträngigen Enden mit dem Klenow-Fragment aufgefüllt und religiert. Dieses modifizierte Plasmid wurde dann mit PstI und HindIII verdaut und P₂ plus RBS zwischen diesen Restriktionsstellen einligiert (pUC18-P₂). Bei dieser Operation verschwindet die ursprüngliche HindIII-Restriktionsstelle des Plasmids und eine neue HindIII-Restriktionsstelle wird eingebaut. pUC18-P₂ wurde dann mit PstI und HindIII verdaut und die DNA der schweren Kette (PstI-HindIII-Insert aus M13) wurde in diese beiden Stellen ligiert (pUC18-HP₂). Dieses Plasmid wurde dann mit EcoRV und BamHI verdaut und die DNA der leichten Kette (EcoRV-BamHI-Insert aus M13) wurde hineinligiert (pUC18-HP₂L).

Das Insert PstI-BamHI wurde dann in pUC18 umkloniert, nachdem dort zuvor die Restriktionsstellen für HindIII, BanII und KpnI entfernt wurden. Die HindIII-Restriktionsstelle wurde wie oben bei pKK233-2 entfernt, wobei die Religation aber ohne Insertion von BamHI Linkern erfolgte. Anschließend wurde das resultierende Plasmid mit SmaI und BanII verdaut und nach Auffüllen der überstehenden Enden durch T4 DNA Polymerase religiert. Die Insertion des PstI-BamHI Restriktionsfragments ergibt pUC-HP₂L. In einer bevorzugten Ausführungsform wurde zusätzlich in den Restriktionsstellen BanII und HindIII eine "TAG"-Sequenz inseriert (Tab. 3). Die "TAG"-Sequenz kodiert für die Erkennungssequenz Glu-Gly-Glu-Glu-Phe des monoklonalen Antikörpers Yl 1/2 (Wehland et al., (1984), EMBO J. 3, 1295). Durch diesen "Peptidmarker" ist das Expressionsprodukt des resultierenden Plasmids pUC-HTP₂L bequem nachweisbar.

Für die Insertion von HP₂L bzw. HTP₂L in den Expressionsvektor wurden beide Plasmide mit PstI und BamHI geschnitten und das PstI-BamHI HP₂L-Insert aus pUC-HP₂L bzw. das HTP₂L-Insert aus pUC-HTP₂L wurde in das modifizierte Plasmid pKK233-2-P₁ in diese beiden Restriktionsstellen ligiert. Eine schematische Darstellung des fertigen Expressionsvektors pFMT wird in Tab. 5 gezeigt.

### Beispiel 2: Synthese von Antikörper-DNA mit Zufallssequenzen in hypervariablen Bereichen

Eine Zusammenstellung der synthetisierten Oligonukleotiden für die Synthese der variablen Teilen von Antikörper-DNA wird in Tab. 1 gezeigt. Für die Synthese der hypervariablen Regionen wurden annähernd zufällig ("random") Nukleotidsequenzen verwendet. Einschränkungen der Zufälligkeit werden in Tab. 1 erläutert. Zwei verschiedene Sätze von Oligonukleotiden wurden synthetisiert. In Satz A sind die hypervariablen Bereiche weitgehend zufällig außer den wenigen Positionen, wo fast ausschließlich bestimmte Aminosäuren vorkommen. In Satz B wurde die Zufälligkeit der Nukleotidsequenzen in den Positionen, wo überwiegend ein oder wenige Aminosäuren vorkommen, zusätzlich eingeschränkt.

Die Oligonukleotide wurden durch HPLC-Chromatographie oder Polyacrylamidgelelektrophorese gereinigt und anschließend 5'-phosphoryliert.

### Beispiel 3: Ligation der synthetisierten Oligonukleotide

Die Oligonukleotide in Tab. 1 wurden schrittweise auf einem Antikörper-DNA-"Template" zusammenligiert. Dafür wurde einzelsträngige M13mpl8-DNA mit den Antikörper-DNA-Inserts in größeren Mengen (etwa lmg) isoliert. Um die Antikörper-DNA vom Vektor zu trennen, wurden die Inserts an den beiden Enden mit zwei passenden Oligonukleotiden doppelsträngig gemacht und mit den Enzymen PstI und HindIII (schwere Kette) bzw. mit EcoRV und BanHI (leichte Kette) verdaut. Die Antikörper-DNA wurde dann auf einer Agargelelektrophorese gereinigt.

Auf diesen DNA "Templates" wurden erst nur drei Oligonukleotide ligiert: H1, pH2 und pH3 (schwere Kette) und L1, pL2 und pL3 (leichte Kette), wobei H1 und L1 an ihrem 5'-Ende mit ³²P zuerst markiert wurden ("p" bedeutet 5'-phosphoryliert). 100pmol Mengen von jedem Oligonukleotid wurden eingesetzt. Die hybridisierten Oligonukleotide wurden auf 2%igem Agarosegelen gereinigt und auf einem Sequenzgel analysiert. Die Menge wurde durch eine Radioaktivitätsmessung ermittelt. Gleiche Molmengen von pH4 und pH5 (schwere Kette) und pL4 und pL5 (leichte Kette) wurden dann an die schon ligierten Oligonukleotide auf dem jeweiligen "Template" ligiert. Diese DNA's wurden dann wie im vorhergehenden Schritt gereinigt und die Prozedur wurde mit gleichen Molmenge von pH6 und pH7 bis zum Reinigungsschritt wiederholt. Die ligierten Oligonukleotide wurden schließlich über ein denaturierendes Polyacrylamidgel gereinigt und vorzugsweise mit der Polymerase-Ketten-Reaktion (PCR) amplifiziert. Alternativ bzw. um Verluste durch den letzten Reinigungsschritt zu vermeiden, wurden die Oligonukleotide direkt nach dem letzten Ligationsschritt mit PCR amplifiziert. Für die PCR wurden die "Primer" H1 und H8 (schwere Kette) bzw. L1 und L6 (leichte Kette) unter Standardbedingungen verwendet. Amplifizierte "Template" DNA wurde selektiv verdaut mit KpnI (leichte Kette) bzw. mit AluI (schwere Kette). Gegebenenfalls wurde ein zweiter Amplifizierungsschritt mit der PCR nachgeschaltet.

### Beispiel 4: Insertion der Antikörper-DNA in das Expressionsplasmid

Die synthetisierte Antikörper-DNA wurde mit den Restriktionsenzymen PstI und BanII (schwere Kette) bzw. BstEII und KpnI (leichte Kette) geschnitten. Die Banden mit dem erwarteten Molekulargewicht wurden durch Agargelelektrophorese gereinigt, mit Ethanol gefällt und anschließend in den ebenso geschnittenen und gereinigten pUC-HP₂L (siehe oben) in zwei Schritten (erst die DNA der leichten Kette und dann die DNA der schweren Kette) ligiert. Das HP₂L-Insert wurde dann in die Restriktionsstellen PstI und BamHI des Plasmids pKK233-2-P₁ (siehe Beispiel 1) ligiert. Entsprechendes gilt für das HTP₂L-Fragment. Die Antikörper-Bibliothek ist somit in dem Antikörper-Expressionsplasmid etabliert (Tab. 6). Der Grund für die Zwischenklonierung in pUC besteht darin, daß die Anwesenheit von einzelnen Teilen des Antikörper-Operons in dem Expressionsvektor das Wachstum des bakteriellen Wirts beeinträchtigt (siehe auch oben).

### Beispiel 5: Expression und "Screenen" von Antikörpern in E.coli

Kompetente E.coli werden mit pFMT-Plasmiden, die die insertierte Antikörper-DNA-Bibliothek enthalten, transfiziert, auf Agaroseplatten aufgezogen und dann mit Nitrozellulosefiltern, die mit dem gesuchten Antigen beschichtet sind, inkubiert. Nach der Entfernung unspezifisch gebundener Antikörper werden die aktiven Klone mit einem markierten Antikörper gegen die aus E.coli sezernierten menschlichen Immunglobuline identifiziert. In der bevorzugten Ausführungsform wird dafür der Antikörper YL 1/2 verwendet, der gegen die "TAG"-Sequenz gerichtet ist.

### Legende zu Fig. 1:

Restriktionskarte des Expressionsvektors pKK233-2 (Amann und Brosius, a.a.O.).
Ptrc bedeutet hybrider Tryptophan-lac Promotor
RBS bedeutet Ribosomenbindungsstelle
rrnB bedeutet ribosomales RNA B Operon
5S bedeutet Gen für 5S RNA

Vor der Klonierung von Antikörper-DNA in den Expressionsvektor wurden die folgenden Änderungen durchgeführt:
**1)** Die SalI und EcoRI Restriktionsstellen wurden zusammen mit der zwischenliegenden DNA entfernt.
**2)** Die HindIII Restriktionsstelle wurde in eine BamHI Restriktionsstelle umgewandelt.

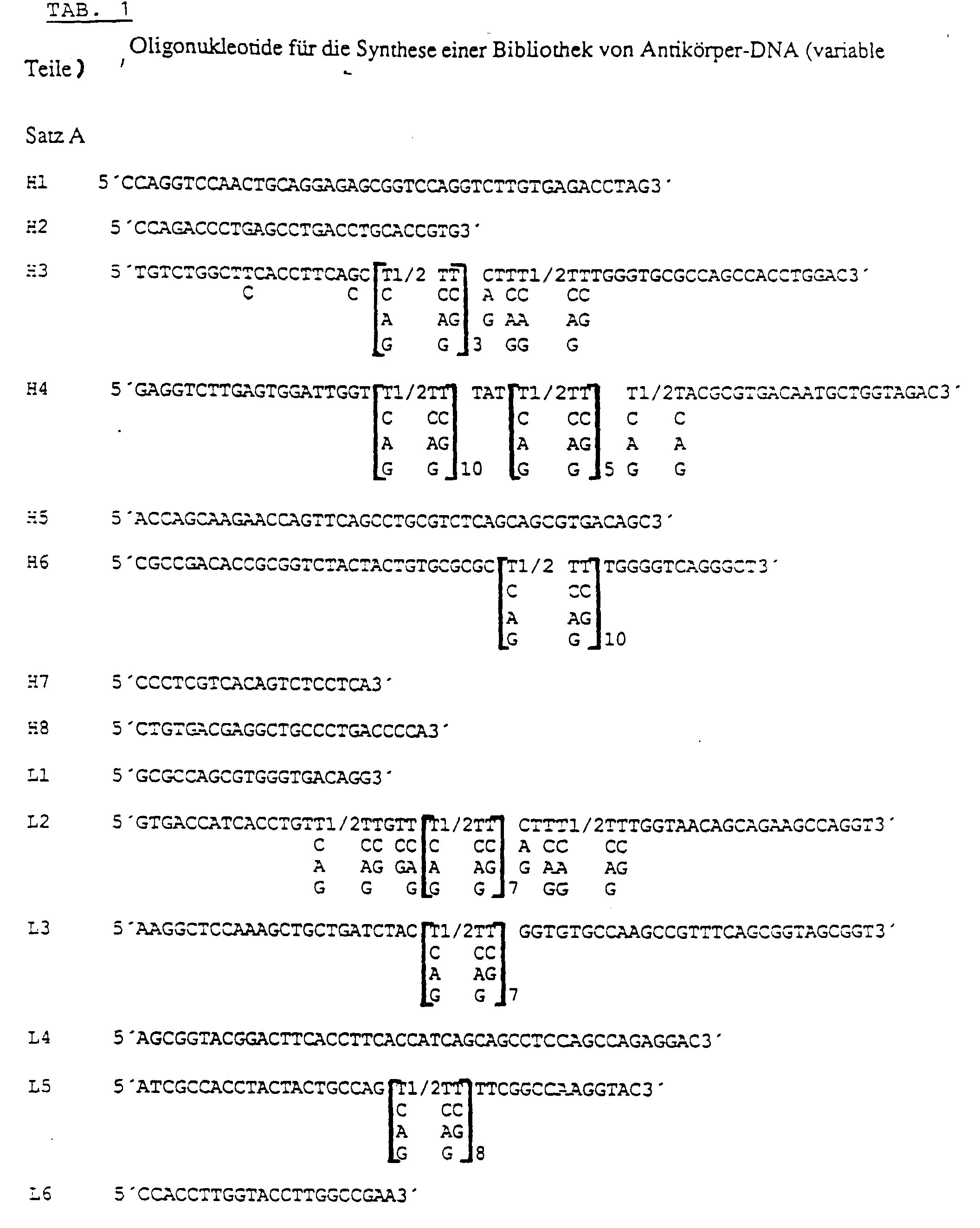

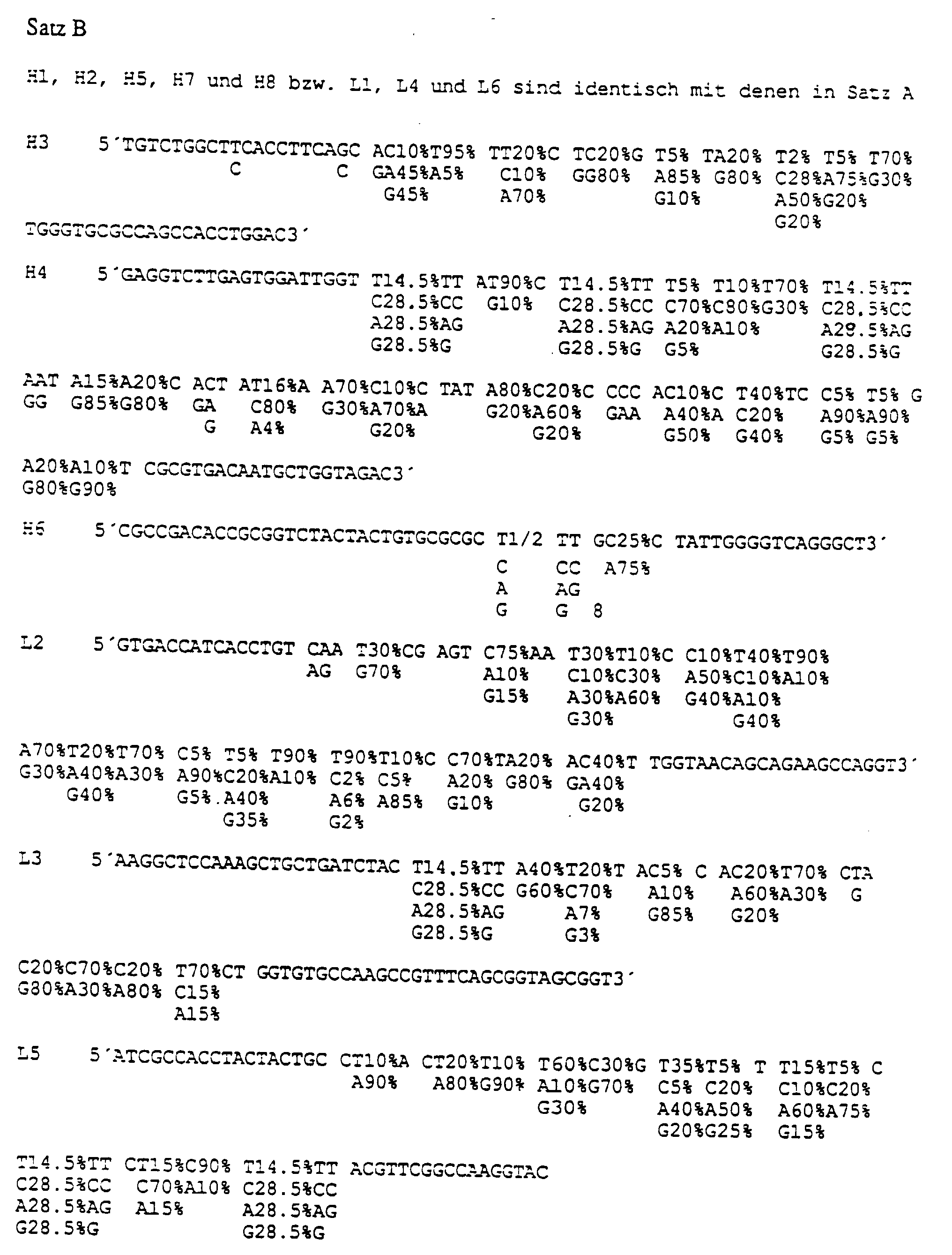

### Erläuterungen zu Tab. 1

Die Zufälligkeit der Satz B Oligonukleotide wurde in einer Weise eingeschränkt, die annähernd die relative Menge von häufigen Aminosäuren in jeder Position der hypervariablen Regionen erzeugt (nach den Tabellen von Kabat et al., a.a.O.). Bei dieser Strategie wurde auch die Zahl der erwarteten Stopcodons noch weiter reduziert. Eine Restriktionsstelle für MluI wurde im Gegensatz zu den Satz A-Oligonukleotiden nicht eingeführt.

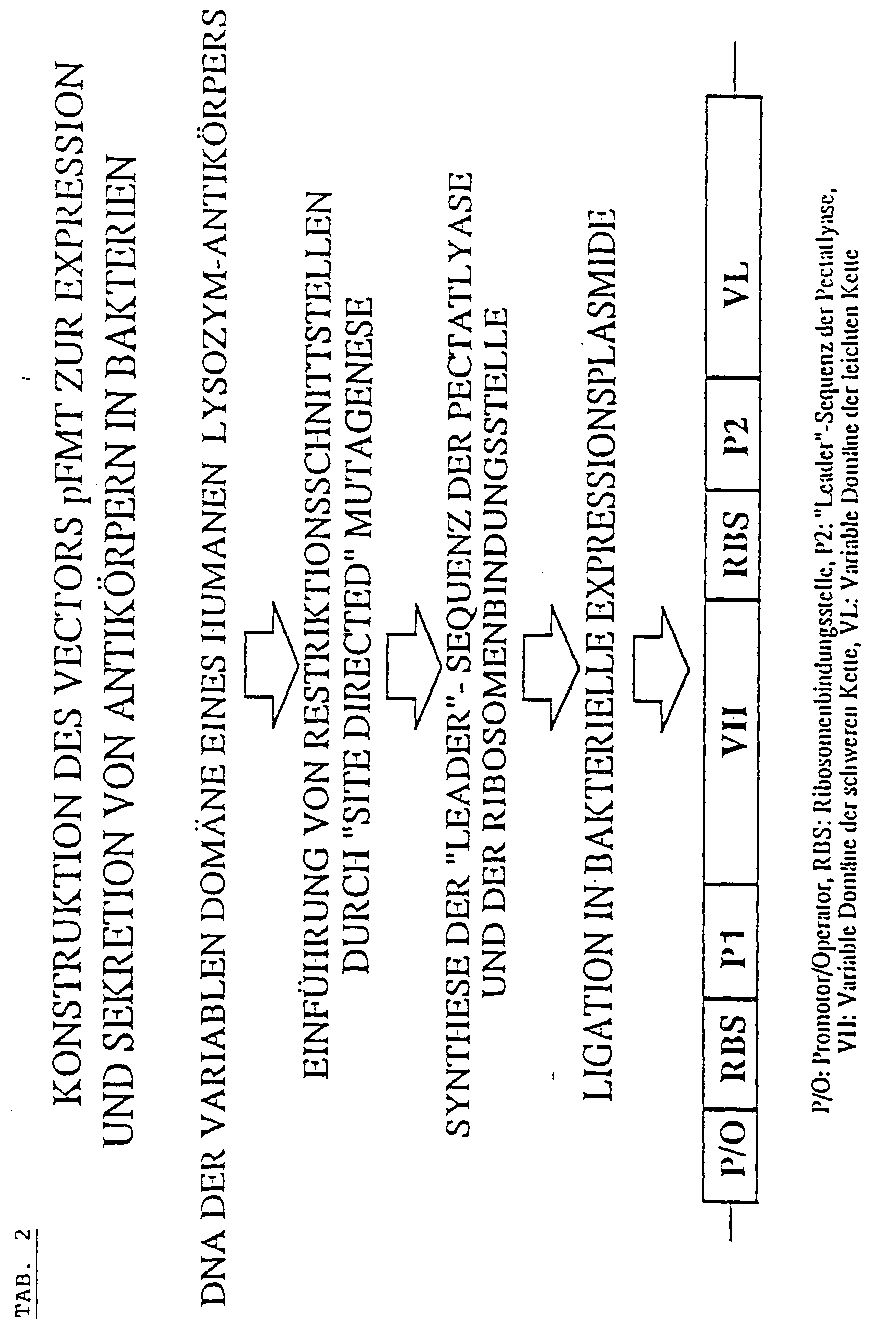

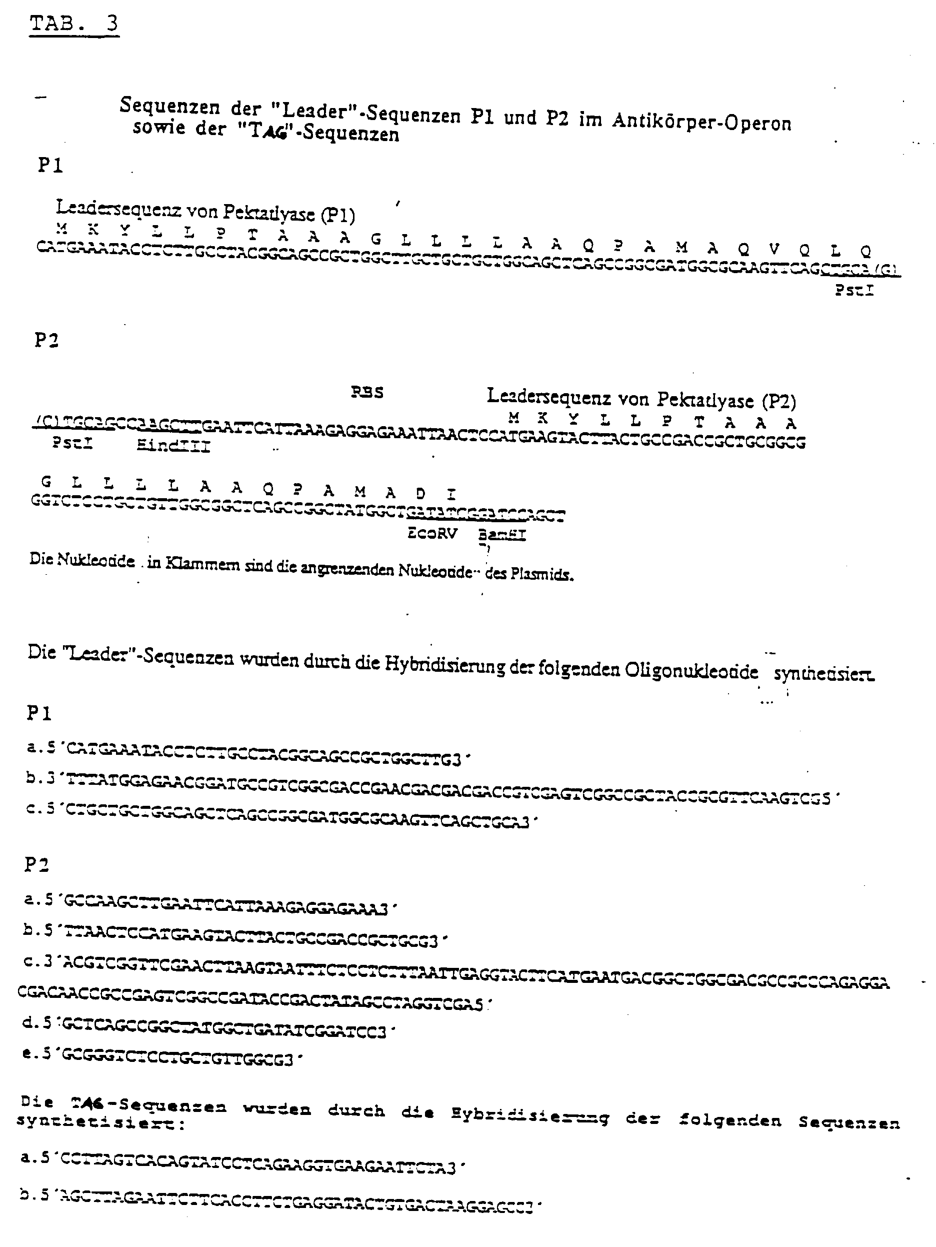

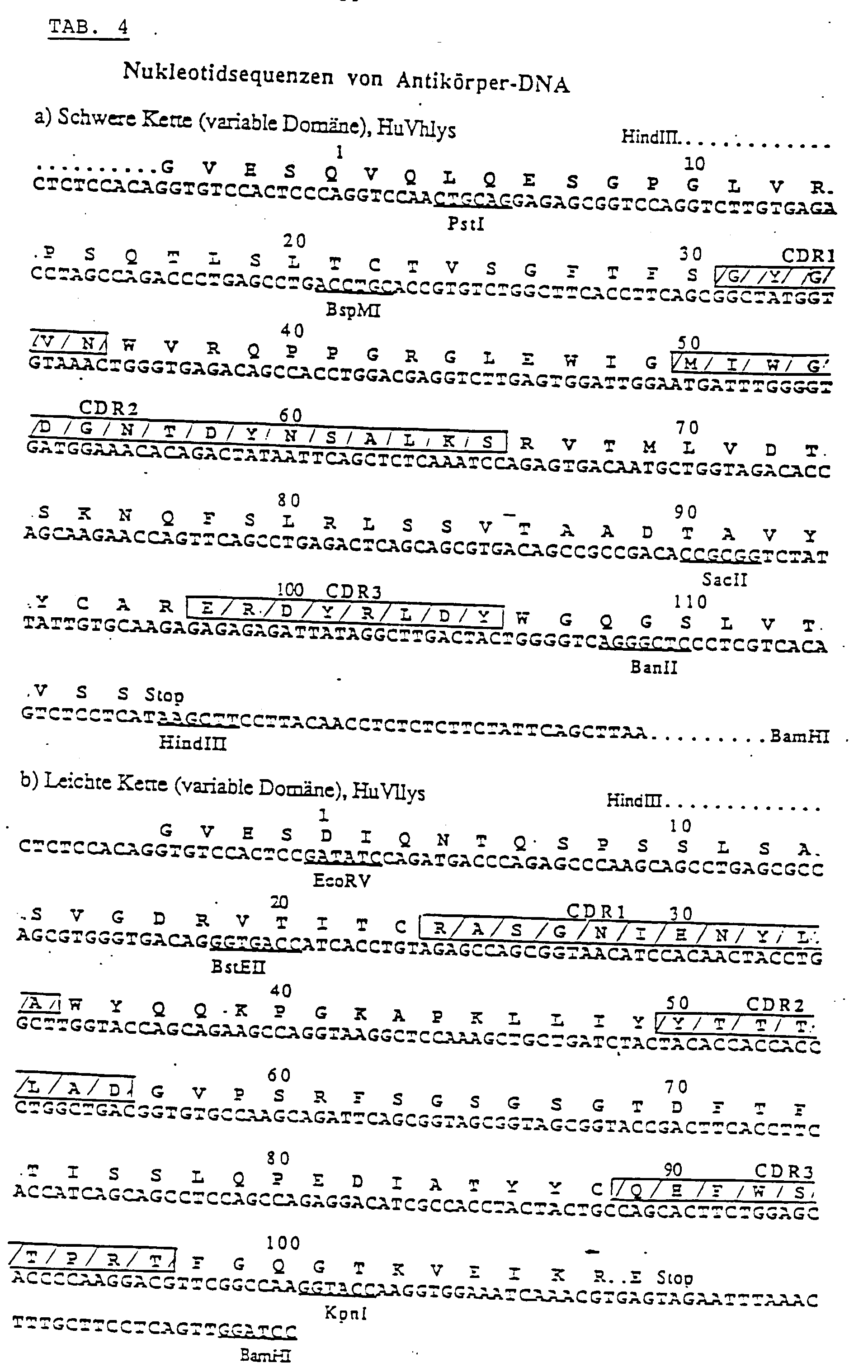

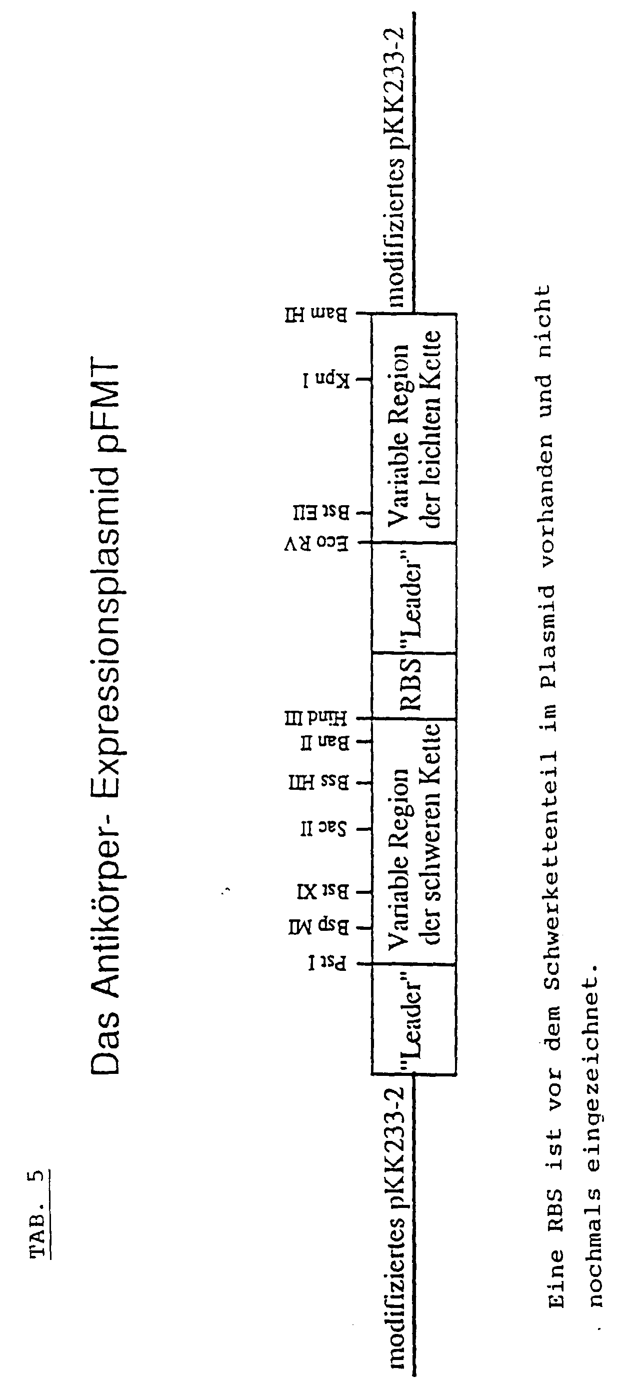

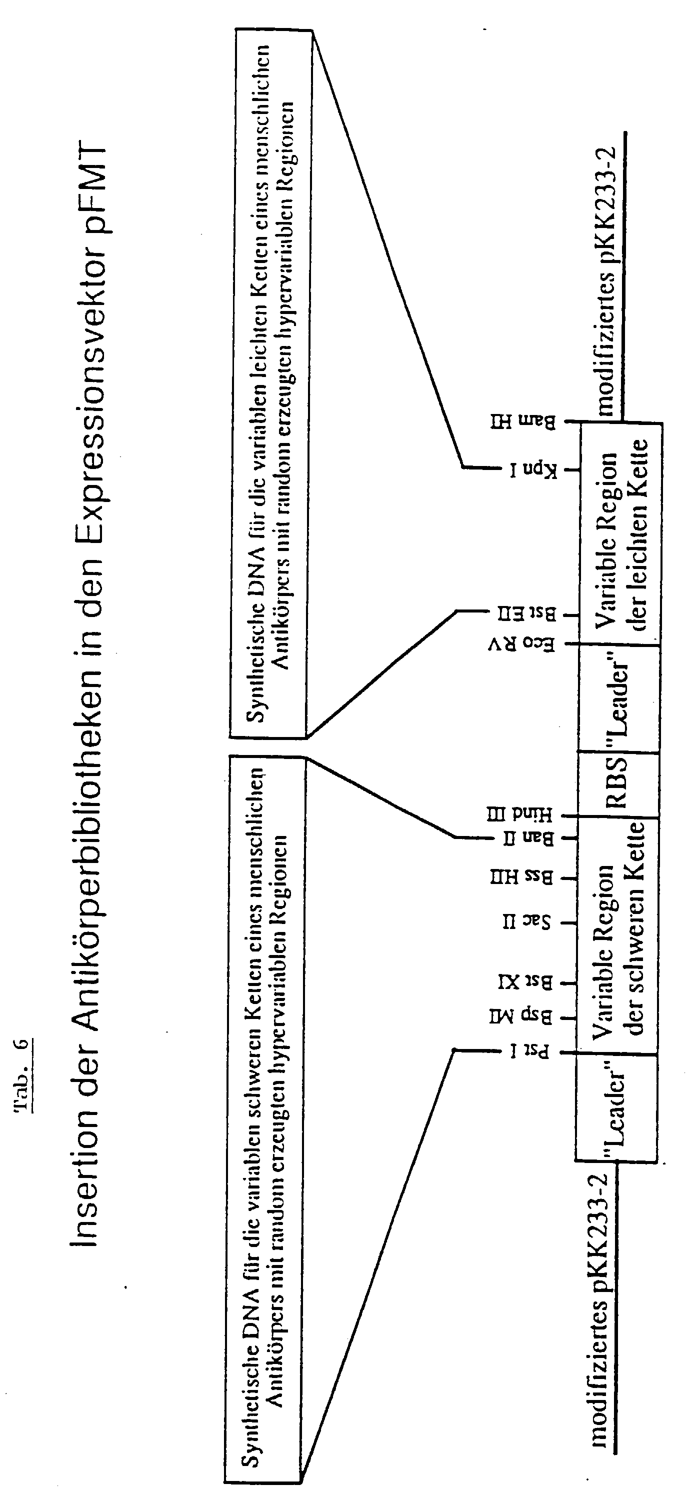

## Patentansprüche (Patentansprüche für folgende(n) Vertragsstaat(en): AT, BE, CH, DE, DK, FR, GB, GR, IT, LI, LU, NL, SE)

1. Synthetische Human-Antikörper-Bibliothek, erhältlich durch
(1) Generierung von Zufallssequenzen für die hypervariablen Bereiche der schweren und leichten Kette, enthaltend die Sequenzen gemäß Tabelle 1, Satz A oder Satz B, wobei die Sequenzen den Einschränkungen unterliegen, daß
(a) an der jeweils ersten Position eines Codons von T nur die Hälfte der für C, G oder A erlaubten Menge zugelassen und daß A in der jeweils dritten Position eines Codons nicht zugelassen ist und
(b) für das zweite Codon innerhalb der CDR1-Region der leichten Kette nur Tripletts zugelassen sind, die für die Aminosäuren V, A oder G kodieren und
(c) für Codon Nr. 10 innerhalb der CDR1-Region der leichten Kette und Codon Nr. 4 innerhalb der CDR1-Region der schweren Kette nur Tripletts zugelassen sind, die für die Aminosäuren V, I oder M kodieren und
(d) für das erste Codon innerhalb der CDR3-Region der leichten Kette nur Tripletts zugelassen sind, die für die Aminosäure Q kodieren und
(e) für Codon Nr. 11 innerhalb der CDR2-Region der schweren Kette nur Tripletts zugelassen sind, die für die Aminosäure Y kodieren und
(2) anschließenden Einbau besagter Zufallssequenzen für die hypervariablen Bereiche in einen Expressionsvektor.

2. Synthetische Human-Antikörper-Bibliothek nach Anspruch 1, dadurch gekennzeichnet, daß die generierten Zufallssequenzen für die hypervariablen Bereiche vor dem Einbau in einen Expressionsvektor amplifiziert werden.

3. Synthetische Antikörper-Bibliothek nach Anspruch 1 oder 2, dadurch gekennzeichnet, daß als Expressionsvektor der Vektor pFMT gemäß Tabelle 5 eingesetzt wird.

4. Verfahren zur Herstellung einer synthetischen Human-Antikörper-Bibliothek gemäß Anspruch 1, dadurch gekennzeichnet, daß für die hypervariablen Bereiche Zufallssequenzen enthaltend die Sequenzen gemäß Tabelle 1, Satz A oder Satz B unter den Einschränkungen (a) bis (e) erzeugt und anschließend in einen Expressionsvektor eingebaut werden.

5. Verfahren nach Anspruch 4, dadurch gekennzeichnet, daß die erzeugten Zufallssequenzen vor Einbau in einen Expressionsvektor amplifiziert werden.

6. Verfahren nach Anspruch 4 oder 5, dadurch gekennzeichnet, daß als Expressionsvektor der Vektor pFMT gemäß Tabelle 5 eingesetzt wird.

7. Verfahren zur Isolierung von Klonen, die spezifische humane Antikörper sezernieren, dadurch gekennzeichnet, daß eine synthetische Human-Antikörper-Bibliothek nach Anspruch 1, 2 oder 3 mit spezifischen Antigenen durchsucht werden.

8. Verfahren nach Anspruch 7, dadurch gekennzeichnet, daß zusätzlich das Markerpeptid Glu-Gly-Glu-Glu-Phe eingebaut wird und die gesuchten Klone mit Antikörpern gegen das Markerpeptid, vorzugsweise mit dem Antikörper YL 1/2 (Wehland et al., Embo J. 3, p. 1295, 1984) identifiziert werden.

9. Verwendung einer synthetischen Human-Antikörper-Bibliothek nach Anspruch 1 oder 2 zur Isolierung von Klonen, die spezifische Antikörper sezernieren.

## Patentansprüche (Patentansprüche für folgende(n) Vertragsstaat(en): ES)

1. Verfahren zur Herstellung synthetischer Human-Antikörper-Bibliotheken, dadurch gekennzeichnet, daß für die hypervariablen Bereiche Zufallssequenzen unter den Einschränkungen (a) bis (e), Satz A oder Satz B gemäß Tab. 1 erzeugt und anschließend in einen Expressionsvektor eingebaut werden.

2. Verfahren nach Anspruch 1, dadurch gekennzeichnet, daß die erzeugten Zufallssequenzen vor Einbau in einen Expressionsvektor amplifiziert werden.

3. Verfahren nach Anspruch 1 oder 2, dadurch gekennzeichnet, daß als Expressionsvektor der Vektor pFMT eingesetzt wird.

4. Verfahren zur Isolierung von Klonen, die spezifische humane Antikörper sezernieren, dadurch gekennzeichnet, daß synthetische Human-Antikörper-Bibliotheken hergestellt nach Anspruch 1 bis 3 mit spezifischen Antigenen durchsucht werden.

5. Verfahren nach Anspruch 4, dadurch gekennzeichnet, daß zusätzlich ein Markerpeptid, vorzugsweise die "TAG"-Sequenz eingebaut wird und die gesuchten Klone mit Antikörpern gegen das Markerpeptid, vorzugsweise mit dem Antikörper YL 1/2 (Wehland et al., Embo J. 3, p. 1295, 1984) identifiziert werden.

## Claims (Claims for the following Contracting State(s): AT, BE, CH, DE, DK, FR, GB, GR, IT, LI, LU, NL, SE)

1. A synthetic human antibody library obtainable by
(1) generation of random sequences for the hypervariable regions of the heavy and light chain, comprising the sequences according to Table 1, set A or set B, where the sequences are subject to the limitations that
(a) at the first position of a codon for T in each case only half of the amount allowed for C, G or A is permitted, and that A is not permitted in the third position of a codon in each case, and
(b) only triplets which code for the amino acids V, A or G are permitted for the second codon within the CDR1 region of the light chain, and
(c) only triplets which code for the amino acids V, I or M are permitted for codon No. 10 within the CDR1 region of the light chain and codon No. 4 within the CDR1 region of the heavy chain, and
(d) only triplets which code for the amino acid Q are permitted for the first codon within the CDR3 region of the light chain, and
(e) only triplets which code for the amino acid Y are permitted for codon No. 11 within the CDR2 region of the heavy chain, and
(2) subsequent incorporation of said random sequences for the hypervariable regions into an expression vector.

2. A synthetic human antibody library as claimed in claim 1, wherein the generated random sequences for the hypervariable regions are amplified before the incorporation into an expression vector.

3. A synthetic antibody library as claimed in claim 1 or 2, wherein the vector pFMT according to Table 5 is used as expression vector.

4. A process for preparing a synthetic human antibody library as claimed in claim 1, which comprises generating random sequences, comprising the sequences according to Table 1, set A or set B, for the hypervariable regions with the limitations (a) to (e), and then incorporating them into an expression vector.

5. The process as claimed in claim 4, wherein the generated random sequences are amplified before incorporation into an expression vector.

6. The process as claimed in claim 4 or 5, wherein the vector pFMT according to Table 5 is used as expression vector.

7. A process for isolating clones which secrete specific human antibodies, which comprises screening a synthetic human antibody library as claimed in claim 1, 2 or 3 using specific antigens.

8. The process as claimed in claim 7, wherein the marker peptide Glu-Gly-Glu-Glu-Phe is additionally incorporated and the desired clones are identified using antibodies against the marker peptide, preferably using the antibody YL 1/2 (Wehland et al., Embo J. 3, p. 1295, 1984).

9. The use of a synthetic human antibody library as claimed in claim 1 or 2 for isolating clones which secrete specific antibodies.

## Claims (Claims for the following Contracting State(s): ES)

1. A process for preparing synthetic human antibody libraries, which comprises generating random sequences for the hypervariable regions with the limitations (a) to (e), set A or set B according to Tab. 1, and then incorporating them into an expression vector.

2. The process as claimed in claim 1, wherein the generated random sequences are amplified before incorporation in an expression vector.

3. The process as claimed in claim 1 or 2, wherein the vector pFMT is used as expression vector.

4. A process for isolating clones which secrete specific human antibodies, which comprises screening synthetic human antibody libraries prepared as claimed in claims 1 to 3 using specific antigens.

5. The process as claimed in claim 4, wherein a marker peptide, preferably the TAG sequence, is additionally incorporated and the desired clones are identified using antibodies against the marker peptide, preferably using the antibody YL 1/2 (Wehland et al., Embo J. 3, p. 1295, 1984).

## Revendications (Revendications pour l'(les) Etat(s) contractant(s) suivant(s): AT, BE, CH, DE, DK, FR, GB, GR, IT, LI, LU, NL, SE)

1. Banque d'anticorps synthétiques humains, obtenue par
(1) génération de séquences aléatoires pour les domaines hypervariables des chaînes lourdes et légères contenant les séquences selon le tableau 1, série A ou série B, les séquences étant soumises aux restrictions suivantes :
(a) à la première position de chaque codon, la quantité autorisée pour T n'est que la moitié de celle de C, G ou A et A n'est pas autorisé à la troisième position de chaque codon,
(b) pour le deuxième codon à l'intérieur de la région CDR1 de la chaîne légère, seuls sont autorisés les triplets qui codent pour les aminoacides V, A ou G,
(c) pour le codon n° 10 à l'intérieur de la région CDR1 de la chaîne légère et pour le codon n° 4 à l'intérieur de la région CDR1 de la chaîne lourde, seuls sont autorisés les triplets qui codent pour les aminoacides V, I ou M,
(d) pour le premier codon à l'intérieur de la région CDR3 de la chaîne légère, seuls sont autorisés les triplets qui codent pour l'aminoacide Q et
(e) pour le codon n° 11 à l'intérieur de la région CDR2 de la chaîne lourde, seuls sont autorisés les triplets qui codent pour l'aminoacide Y et
(2) finalement introduction desdites séquences aléatoires pour les domaines hypervariables dans un vecteur d'expression.

2. Banque d'anticorps synthétiques humains selon la revendication 1, caractérisée en ce que des séquences aléatoires générées pour les domaines hypervariables sont amplifiées avant leur introduction dans un vecteur d' expression.

3. Banque d'anticorps synthétiques selon la revendication 1 ou 2, caractérisée en ce que le vecteur pFMT selon le tableau 5 est utilisé comme vecteur d'expression.

4. Procédé pour la préparation d'une banque d'anticorps synthétiques humains selon la revendication 1, caractérisé en ce que les séquences aléatoires générées pour les domaines hypervariables contenant les séquences selon le tableau 1, série A ou série B, sont produites en tenant compte des restrictions (a) à (e) et finalement introduites dans un vecteur d'expression,

5. Procédé selon la revendication 4, caractérisé en ce que les séquences aléatoires générées sont amplifiées avant leur introduction dans un vecteur d'expression.

6. Procédé selon la revendication 4 ou 5, caractérisé en ce que le vecteur pFMT selon le tableau 5 est utilisé comme vecteur d'expression.

7. Procédé pour l'isolement de clones qui produisent des anticorps humains spécifiques, caractérisé en ce qu'une banque d'anticorps synthétiques humains selon la revendication 1, 2 ou 3 est testée avec des antigènes spécifiques.

8. Procédé selon la revendication 7, caractérisé en ce que le peptide marqueur Glu-Gly-Glu-Glu-Phe est introduit en plus et les clones cherchés sont identifiés avec des anticorps dirigés contre le peptide marqueur, de préférence avec l'anticorps YL 1/2 (Wehland et al., Embo J. 3, p. 1295, 1984).

9. Utilisation d'une banque d'anticorps synthétiques humains selon la revendication 1 ou 2 pour l'isolement de clones qui produisent des anticorps spécifiques.

## Revendications (Revendications pour l'(les) Etat(s) contractant(s) suivant(s): ES)

1. Procédé de préparation de banque d'anticorps synthétiques humains, caractérisé en ce que des séquences aléatoires pour les domaines hypervariables sont générées en respectant les restrictions (a) à (e), série A ou série B selon le tableau 1, puis introduites dans un vecteur d'expression.

2. Procédé selon la revendication 1, caractérisé en ce que les séquences aléatoires générées sont amplifiées avant introduction dans un vecteur d'expression.

3. Procédé selon la revendication 1 ou 2, caractérisé en ce que l'on utilise le vecteur pFMT comme vecteur d'expression.

4. Procédé pour l'isolement de clones qui produisent des anticorps humains spécifiques, caractérisé en ce que des banques d'anticorps synthétiques humains, préparés selon les revendications 1 à 3, sont testées avec des antigènes spécifiques.

5. Procédé selon la revendication 4, caractérisé en ce qu'un peptide marqueur, de préférence la séquence "TAG" est introduit en plus et les clones cherchés sont identifiés avec des anticorps dirigés contre le peptide marqueur, de préférence avec l'anticorps YL 1/2 (Wehland et al., Embo J. 3, p. 1295, 1984).
